# EUROPEAN PATENT SPECIFICATION

(11) **EP 2 231 603 B1**
(45) Date of publication and mention of the grant of the patent: **01.05.2013**
(21) Application number: 07873379.7
(22) Date of filing: 18.12.2007
(51) Int. Cl.: C07D 213/65, A61K 31/44, A61P 3/00

(54) **SUBSTITUTED 3 -HYDROXYPYRIDINES AND PHARMACEUTICAL COMPOSITIONS THEREOF**
SUBSTITUIERTE 3-HYDROXYPYRIDINE UND PHARMAZEUTISCHE ZUSAMMENSETZUNGEN DAVON
3-HYDROXYPYRIDINES SUBSTITUÉES ET COMPOSITIONS PHARMACEUTIQUES ASSOCIÉES

(43) Date of publication of application: 29.09.2010
(73) Proprietor: Biryukov, Dmitri Valerievich, Leningradskaya obl. 187100 (RU); Povazhnyy, Dmitriy Borisovich, Moscow, 123060 (RU); Pomytkin, Igor Anatolievich, Moscow, 123557 (RU)
(72) Inventor: Biryukov, Dmitri Valerievich, Leningradskaya obl., 187100 (RU); Pomytkin, Igor Anatolievich, Moscow, 123557 (RU)
(74) Representative: Hayes, Adrian Chetwynd
(86) International application number: PCT/RU2007/000715
(87) International publication number: WO 2009/078746

(56) References cited:
- WO-A-2005/077902
- DATABASE CHEMCATS [Online] CHEMICAL ABSTRACTS SERVICE, COLUMBUS, OHIO, US; XP002500018 retrieved from STN & TIMTEC OVERSEAS STOC: 20 November 2007 (2007-11-20), TIMTEC INC. , NEWARK, DE, USA
- WEBER ET AL.: "Synthese und Photoisomerisierung von sterisch gehinderten 2,6-Dialkylpyridin-N-oxiden" ZEITSCHRIFT FÜR NATURFORSCHUNG B, vol. 45, no. 5, 1990, pages 701-706, XP009107210
- DATABASE WPI Thomson Scientific, London, GB; AN 2003-013789 XP002500134 INCHINA ET AL.: "Treatment of atherosclerosis of lower limbs by injection of 2-ethyl-6-methyl-3-oxypyridine succinate" & RU 2 190 404 A (INCHINA ET AL.) 10 October 2002 (2002-10-10) cited in the application & RU 2 190 404 A (INCHINA ET AL.) 10 October 2002 (2002-10-10)

## Description

### Field of the Invention

The present invention relates to novel therapeutic compounds and pharmaceutically acceptable salts thereof, pharmaceutical compositions containing the same, the compounds for use as medicaments, and use of the compounds for the manufacture of specific medicaments. The novel compounds are useful for the treatment of age-related disorders accompanied with dysfunctional insulin receptor signaling.

### Background of the invention

3-Hydroxypiridines are a class of drugs available on a market. RU Patents No. 2168992, 2168993, 2185826, and 2190404 disclose 2-ethyl-6-methyl-3-hydroxypyridine useful for treating arthritis, ischemia, metabolic syndrome, and atherosclerosis.

Because of hydrophilic property, 2-ethyl-6-methyl-3-hydroxypyridine has limited transport capacity to nervous tissues and brain. Thus, it is desirable to develop novel 3-hydroxypyridines with increased lipophilicity.

It is an object of the present invention to provide novel substituted 3-hydroxypyridines, or pharmaceutically acceptable salts thereof, and pharmaceutical compositions thereof.

### Detailed Description of the Invention

The present invention provides a compound of formula **(I)** 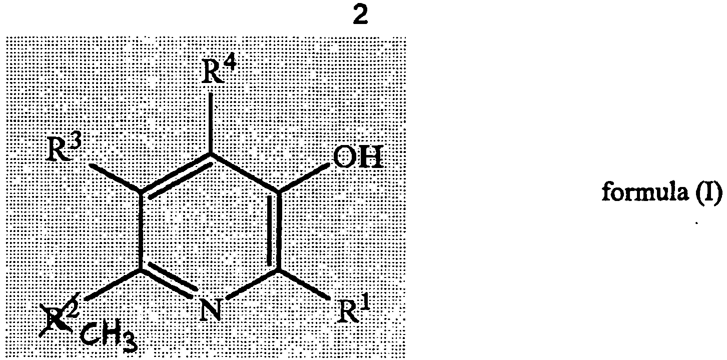 wherein
R¹ is selected from the group consisting of C₂₋₈alkyl,
R³ is independently selected from the group consisting of H and C₁₋₈alkyl,
R⁴ is independently selected from the group consisting of C₁₋₈alkyl,
or a pharmaceutically acceptable salt thereof.

The term «pharmaceutically acceptable salt" refers to non-toxic acid addition salts. The pharmaceutically acceptable salts of the invention are prepared by a reaction of compound of formula (I) with a pharmaceutically acceptable acid by methods well-known from the art. Such salts include, but are not limited to, hydrochloride, hydrobromide, succinate, fumarate, malate, and acetate salt. Preferably, the pharmaceutically acceptable salt of the invention is selected from the group consisting of hydrochloride salt, succinate salt, fumarate salt, L-malate salt, ketoglutarate salt, and citrate salt.

The term "C₂₋₈alkyl" as used herein at all occurrences means a straight or branched chain radical of 2 to 8 carbon atoms, unless the chain length is limited thereto, including, but not limited to ethyl, n-propyl, isopropyl, n-butyl, isobutyl and t-butyl, pentyl, n-pentyl, isopentyl, neopentyl, hexyl, and octyl and the simple aliphatic isomers thereof.

The term "C₁₋₈alkyl" as used herein at all occurrences means a straight or branched chain radical of 1 to 8 carbon atoms, unless the chain length is limited thereto, including, but not limited to methyl, ethyl, n-propyl, isopropyl, n-butyl, isobutyl and t-butyl, pentyl, n-pentyl, isopentyl, neopentyl, hexyl, and octyl and the simple aliphatic isomers thereof.

Preferred compounds of the present invention include 2-ethyl-4,6-dimethyl-3-hydroxypyridine, succinic acid salt (1:1), and 2-ethyl-4,5,6-trimethyl-3-hydroxypyridine, succinic acid salt (1:1).

Further, the present invention provides a pharmaceutical composition comprising the compound of formula (I) as depicted above
or a pharmaceutically acceptable salt thereof, and a pharmaceutically acceptable carrier.

The term «pharmaceutically acceptable carrier" refers to a one or more compatible solid or liquid filler diluents or encapsulating substances which are suitable for administration to any portion of the body of a mammal, preferably a human.

The compositions of the invention are prepared by methods well-known from the art in accordance with accepted pharmaceutical procedures, for example, as described in Remington's Pharmaceutical Sciences, seventeenth edition, ed. Alfonso R. Gennaro, Mack Publishing Company, Easton, Pa., Eighteenth edition (1990).

The compound of formula (I) or a pharmaceutically acceptable salt thereof according to the invention, in the form of free base or salts with pharmaceutically acceptable acids, or solutions thereof, can be brought into suitable dosage forms, such as compositions for administration through the oral, rectal, transdermal, parenteral, nasal, or pulmonary route in accordance with accepted pharmaceutical procedures. Such pharmaceutical compositions according to the invention comprise the compounds according to the invention in association with compatible pharmaceutically acceptable carrier materials, or diluents, as is well known in the art. The carriers may be any inert material, organic or inorganic, suitable for administration, such as for example: water, gelatin, gum arabicum, lactose, microcrystalline cellulose, starch, sodium starch glycolate, calcium hydrogen phosphate, magnesium stearate, talcum, and colloidal silicon dioxide. Such compositions may also contain other pharmaceutically active agents, and conventional additives such as for example, stabilizers, wetting agents, emulsifiers, flavoring agents, buffers, binders, disintegrants, lubricants, glidants, antiadherents, and propellants. The content of compound of formula (I) or a pharmaceutically acceptable salt thereof is in the range from 0.1 to 99 %, preferably 0.5 to 10 % by the weight of the composition.

The compositions of the invention can be prepared in a variety of unit dosage forms. Such forms are include, but are not limited to, injections, eye drops, spray, gel, ointment, tablet, capsule, slow releasing forms, and powder.

The compound of formula (I) or a pharmaceutically acceptable salt thereof according to the present invention can be administered in therapeutically effective amounts in any suitable way. The compounds according to the invention can be made up in solid or liquid form, such as, for example, tablets, capsules, powders, syrups, elixirs, aerosols, sterile solutions, suspensions or emulsions.

The term "therapeutically effective amount" refers to a nontoxic but sufficient amount of an active agent to provide the desired therapeutic effect. Preferably, the therapeutically effective amount of compounds of formula (I) is from 1 to 500 mg per a unit dosage form of compositions of the present invention. More preferably, from 50 to 150 mg per a unit dosage form.

The dosage of the specific compound according to the invention will vary depending on its potency, the mode of administration, the age and weight of the patient and the severity of the condition to be treated. For example, the medication may be administered orally once or twice daily, or less frequently, or intermittently.

The compounds and compositions of the present invention can be used for treating age-related diseases including metabolic diseases, neurodegenerative diseases, inflammatory disorders, and CNS disorders. Preferably, the compounds and compositions of the present invention are useful for treating a disease, condition or disorder associated with impaired insulin action comprising the step of administering to a mammal in need of such treatment a therapeutically effective amount of a compound of formula (I), a pharmaceutically acceptable salt thereof, wherein said disease, condition or disorder associated with impaired insulin action is selected from the group consisting of diabetes and its complication, Type 2 diabetes, insulin resistance, hyperglycemia, hyperinsulinemia, elevated blood levels of fatty acids or glycerol; hyperlipidemia, obesity, hypertriglyceridemia, dyslipidemia, Syndrome X, atherosclerosis, polycystic ovary syndrome, aging, or metabolic syndrome.

As used herein, the term "treating a disease" means treating, controlling, preventing and/or reducing one or more clinical signs (i.e., symptoms) of the disease in a mammal in need thereof.

Nonexclusive examples of mammals of the invention include humans and companion animals such as cats and dogs. Preferably, the mammal is a human.

Further, the present invention provides a process for preparing the compound of formula (I) as depicted above, comprising the step of reacting a compound of formula (II) 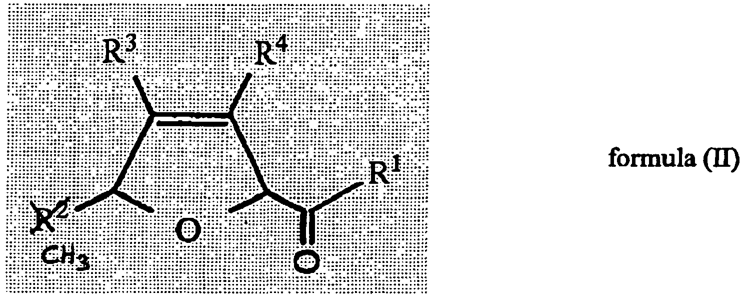 with ammonia, wherein
R¹ is selected from the group consisting of C₂₋₈alkyl,
R³ is independently selected from the group consisting of H and C₁₋₈alkyl,
R⁴ is independently selected from the group consisting of C₁₋₈alkyl.

The starting compounds of formula (II) could be prepared by methods well-known from the art. For example, compounds of formula (II) could be prepared by reaction of well-known from the art alkylsubstituted furans with carboxylic acid anhydrides in the presence of a catalyst, preferably phosphoric acid. The reaction for the preparation of 3-hydroxypyridines from 2-acylfurans is well-known from a literature. P. Bosshard, C.H. Eugster, Adv. Heterocycl. Chem. 7, 377, 1966. In the preferred embodiment of the invention, this step is carried out in absolute ethanol as the solvent for gas ammonia. The reaction is preferably carried out in autoclave at high temperature, but the reaction can be effected also under different conditions. The final pharmaceutical acceptable salt is obtained by reaction of resulting 3-hydroxypyridine with acid in anhydrous medium.

The following examples are presented to demonstrate the invention. The examples are illustrative only and are not intended to limit the scope of the invention in any way.

### Example 1.

### 2-Ethyl-4,5,6-trimethyl-3-hydroxypyridine, succinic acid salt (1:1).

### (1) 2-Propionyl-3,4,5-trimethylfuran

85% Phosphoric acid (0.05 mole) is slowly dropped into a mixture of 2,3,4-dimethylfuran (0.3 mole, CAS registry number [10599-57-2]) and propionic anhydride (78.1 g, 0.6 mole) heated to 40°C. The reaction mixture is heated to 60°C for 2 hours. Temperature is allowed to reach the room's one, then 120 ml of water are added, stirring for 1 more hour. The organic phase is separated and treated with sodium carbonate saturated solution, stirring for 24 hours, to destroy the untreated anhydride and acid. After that, the solution is extracted with chloroform (300 ml x 3), then the combined organic phases are dried with Na₂SO₄ and evaporated to obtain an oily residue which is distilled under vacuum, recovering the fraction boiling at 86-89°C (0.6 mm). The pure ketone is obtained (25.1 g, 49%)

### (2) 2-Ethyl-4,5,6-trimethyl-3-hydroxypyridine

An ammonia saturated solution in absolute ethanol (50 ml), obtained at - 20°C, is placed into an autoclave, then the above prepared ketone (0.36 mole) is added thereto. The reaction mixture is heated to 170°C for 15 hours, with stirring. After cooling, ethanol is evaporated off under reduced pressure to obtain an oily residue which is taken up into a 2N sodium hydroxide solution (400 ml). After stirring and thoroughly triturating, the alkali solution is extracted with chloroform (100 ml x 4) to recover the unreacted ketone. The alkali liquors are neutralized with concentrated hydrochloric acid to separate 2-ethyl-4,5.6-trimethyl-3-hydroxypyridine. Liquors are extracted with chloroform (200 ml x 8), and the organic extracts are washed with some water, dried with Na₂SO₄, filtered and evaporated to give more product. The two solid fractions are combined and repeatedly treated with anhydrous ether (250 ml x 6) to separate the present chloride. From the ether solution, during concentration, the 2-ethyl-4,5,6-trimethyl-3-hydroxypyridine progressively crystallizes (27 g, 61%); Rf - 0.39 (AcOEt).

### (3) Title product

Saturated solution of succinic acid (0.1 mole) in anhydrous ethanol is added to the solution of 2-ethyl-4,5,6-trimethyl-3-hydroxypyridine (0.1 mole) in anhydrous ethanol. Ethanol is evaporated and the product is recristallized from isopropanol-acetone to purity. Elementary analysis for C₁₄H₂₁NO₅ (283.3): calc. % C 59.35, H 7.47, N 4.94; found. % C 59.42, H 7.52, N 4.89. ¹H-NMR analysis confirms the expected structure.

### Example 2.

### 2-Ethyl-4,6-dimethyl-3-hydroxypyridine, succinic acid salt (1:1).

### (1) 2-Propionyl-3,5-dimethylfuran

85% Phosphoric acid (0.05 mole) is slowly dropped into a mixture of 2,4-dimethylfuran (0.3 mole, CAS registry number [3710-43-8]) and propionic anhydride (78.1 g, 0.6 mole) heated to 40°C. The reaction mixture is heated to 60°C for 2 hours. Temperature is allowed to reach the room's one, then 120 ml of water are added, stirring for 1 more hour. The organic phase is separated and treated with sodium carbonate saturated solution, stirring for 24 hours, to destroy the untreated anhydride and acid. After that, the solution is extracted with chloroform (300 ml x 3), then the combined organic phases are dried with Na₂SO₄ and evaporated to obtain an oily residue which is distilled under vacuum, recovering the fraction boiling at 71-75°C (0.6 mm). The pure ketone is obtained (19.6 g, 41%)

### (2) 2-Ethyl-4,6-dimethyl-3-hydroxypyridine

An ammonia saturated solution in absolute ethanol (50 ml), obtained at - 15°C, is placed into an autoclave, then the above prepared ketone (0.36 mole) is added thereto. The reaction mixture is heated to 170°C for 15 hours, with stirring. After cooling, ethanol is evaporated off under reduced pressure to obtain an oily residue which is taken up into a 2N sodium hydroxide solution (400 ml). After stirring and thoroughly triturating, the alkali solution is extracted with chloroform (100 ml x 4) to recover the unreacted ketone. The alkali liquors are neutralized with concentrated hydrochloric acid to separate 2-ethyl-4,6-dimethyl-3-hydroxypyridine. Liquors are extracted with chloroform (200 ml x 8), and the organic extracts are washed with some water, dried with Na₂SO₄, filtered and evaporated to give more product. The two solid fractions are combined and repeatedly treated with anhydrous ether (250 ml x 6) to separate the present chloride. From the ether solution, during concentration, the 2-ethyl-4,6-dimethyl-3-hydroxypyridine progressively crystallizes (29 g, 67%); Rf- 0.37 (AcOEt).

### (3) Title product

Saturated solution of succinic acid (0.1 mole) in anhydrous ethanol is added to the solution of 2-ethyl-4,6-dimethyl-3-hydroxypyridine (0.1 mole) in anhydrous ethanol. Ethanol is evaporated and the product is recristallized from isopropanol-acetone to purity. Elementary analysis for C₁₃H₁₉NO₅ (269.3): calc. % C 57.98, H 7.11, N 5.20; found. % C 57.92, H 7.19, N 5.12. ¹H-NMR analysis confirms the expected structure.

### Example 3.

This example demonstrates injection formulation comprising compound of formula (I).

| Ingredient | Content |
|---|---|
| Compound of formula (I) | 200 mg |
| Disodium phosphate USP/Ph Eur | qs to pH 5.5 |
| Water for injections USP/Ph Eur | to 4.0 ml |

Compound of formula (I) is dissolved in water for injection to the desired volume, 0.4M disodium phosphate is added to pH 5.0. In this manner, solution with concentration of compound of formula (I) of 5% is prepared. The solution is filtered through a sterilizing grade filter (0.2 µm), and filled into ampoules.

### Example 4.

This example demonstrates injection formulation comprising 2-Ethyl-4,6-dimethyl-3-hydroxypyridine, succinic acid salt (1:1).

| Ingredient | Content |
|---|---|
| 2-Ethyl-4,6-dimethyl-3-hydroxypyridine, succinic acid salt (1:1) | 100 mg |
| | qs to pH 5.5 |
| Disodium phosphate USP/Ph Eur | to 4.0 ml |
| Water for injections USP/Ph Eur | |

2-Ethyl-4,6-dimethyl-3-hydroxypyridine, succinic acid salt (1:1) is dissolved in water for injection to the desired volume, 0.4M disodium phosphate is added to pH 5.5. In this manner, solution with concentration of 2-Ethyl-4,6-dimethyl-3-hydroxypyridine, succinic acid salt (1:1), of 5% is prepared. The solution is filtered through a sterilizing grade filter (0.2 µm), and filled into ampoules.

### Example 5.

This example demonstrates injection formulation comprising 2-Ethyl-4,5,6-trimethyl-3-hydroxypyridine, succinic acid salt (1:1).

| Ingredient | Content |
|---|---|
| 2-Ethyl-4,5,6-trimethyl-3-hydroxypyridine, succinic acid salt (1:1) | 100 mg |
| | qs to pH 5.5 |
| Disodium phosphate USP/Ph Eur | to 4.0 ml |
| Water for injections USP/Ph Eur | |

2-Ethyl-4,5,6-trimethyl-3-hydroxypyridine, succinic acid salt (1:1) is dissolved in water for injection to the desired volume, 0.4M disodium phosphate is added to pH 5.5. In this manner, solution with concentration of 2-Ethyl-4,5,6-trimethyl-3-hydroxypyridine, succinic acid salt (1:1), of 5% is prepared. The solution is filtered through a sterilizing grade filter (0.2 µm), and filled into ampoules.

### Example 6.

This example demonstrates efficacy of compounds of the invention for enhancement of activation of insulin receptor.

Human hepatoma HepG2 cells were treated with 5 nM insulin; 50 µM 2-Ethyl-4,5,6-trimethyl-3-hydroxypyridine, succinic acid salt (1:1); 50 µM 2-Ethyl-4,6-dimethyl-3-hydroxypyridine, succinic acid salt (1:1); or their combinations for 10 min in phosphate buffered salt saline containing 5 mM glucose. Activation of insulin receptor in cells was assessed by phosphorylation of tyrosine residues 1162/1163 in the region of the receptor kinase domain with the PhosphoDetect™ insulin receptor (pTyr1162/1163) ELISA kit (Calbiochem). It is generally recognized that the phosphorylation increase activity of receptor to about 200-fold as compared to unphosphorylated one. The results are expressed as a percentage of the response produced to 100 nM insulin and presented in Table 1 as mean ± SEM (n=8).

**Table 1**

| Treatment | Insulin receptor phosphorylation, % |
|---|---|
| Control (none) | 11 ± 3 |
| Insulin, 5 nM | 24 ± 8* |
| 2-Ethyl-4,6-dimethyl-3-hydroxypyridine, succinic acid salt (1:1), 50 µM | 14 ± 4 |
| 2-Ethyl-4,5,6-trimethyl-3-hydroxypyridine, succinic acid salt (1:1), 50 µM | 13 ± 2 |
| Insulin, 5 nM + 2-Ethyl-4,6-dimethyl-3-hydroxypyridine, succinic acid salt (1:1), 50 µM | 56 ± 7*^{#} |
| Insulin, 5 nM + 2-Ethyl-4,5,6-trimethyl-3-hydroxypyridine, succinic acid salt (1:1),50 µM | 62 ± 8*^{#} |

| | |
|---|---|
| *Differs significantly of Control (P<0.05). ^{#} Differs significantly of Insulin 5 nM (P<0.05). | |

Table 1 demonstrates that compounds of the invention significantly enhanced insulin receptor activation.

### Example 7.

This example demonstrates efficacy of compounds of the invention for the treatment of insulin resistance, dyslipidemia, and diabetes.

The streptozotocin (Sigma, St. Louis, MO, USA) solved in citrate buffer (0.05M, pH 5.5) was injected into tail vein of male albino Wistar rats in a dose of 35 mg per kg of animal body weight to induce decompensated insulin resistance. The rats with glucose levels more than 14.0 mmol/l were used in the experiment one week after the streptozotocin injection. Fasting serum glucose concentrations were determined by the glucose oxidase method; plasma insulin concentrations were determined by a double-antibody radioimmunoassay kit; and plasma triglycerides were determined by enzymatic methods.

Streptozotocin-induced rats were assigned to three groups: a control rats (n=10) and experimental rats (n=20). Control rats received daily i.p. injections of saline for 7 days. Experimental rats received daily i.p. injections of 10 mg/kg 2-Ethyl-4,6-dimethyl-3-hydroxypyridine, succinic acid salt (1:1); or 2-Ethyl-4,5,6-trimethyl-3-hydroxypyridine, succinic acid salt (1:1) for 7 days. Biochemical parameters were determined just before start of the treatment and on 14 day from the start of the treatment.

The results are demonstrated in Table 2 as mean ± SEM (n=10).

**Table 2.**

| Treatment | Glucose, mmol/l | |
|---|---|---|
| | Before treatment | 14 Day |
| Control (n=10) | 15.3 ± 2.9 | 13.7 ± 1.2 |
| 2-Ethyl-4,6-dimethyl-3-hydroxypyridine (n=10) | 15.9 ± 2.1 | 8.5 ± 1.3* |
| 2-Ethyl-4,5,6-trimethyl-3-hydroxypyridine (n=10) | 16.0 ± 2.6 | 7.3 ± 1.1* |

| | Insulin, ng/ml | |
|---|---|---|
| | Before treatment | 14 Day |
| Control (n=10) | 10.8 ± 1.4 | 10.3 ± 1.7 |
| 2-Ethyl-4,6-dimethyl-3-hydroxypyridine (n=10) | 11.2 ± 1.7 | 2.9 ± 0.4* |
| 2-Ethyl-4,5,6-trimethyl-3-hydroxypyridine (n=10) | 11.4 ± 2.0 | 2.3 ± 0.5* |

| | Triglycerides, mmol/l | |
|---|---|---|
| | Before treatment | 14 Day |
| Control (n=10) | 3.0 ± 0.3 | 2.7 ± 0.3 |
| 2-Ethyl-4,6-dimethyl-3-hydroxypyridine (n=10) | 2.9 ± 0.4 | 1.8 ± 0.4* |
| 2-Ethyl-4,5,6-trimethyl-3-hydroxypyridine (n=10) | 3.1 ± 0.2 | 1.6 ± 0.3* |

| | | |
|---|---|---|
| *Differs significantly of Control (P<0.05). | | |

Table 2 demonstrates that compounds of the invention are useful for the treatment of diabetes, insulin resistance, and hyperlipidemia.

## Claims

1. A compound of formula (I) 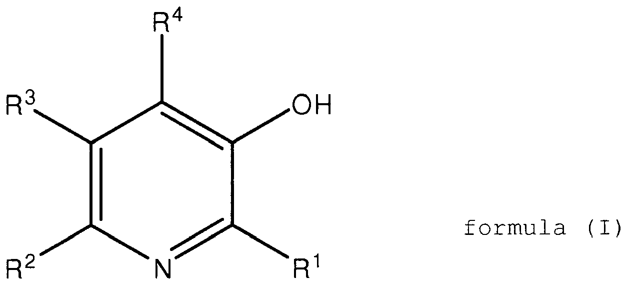 wherein
R¹ is selected from the group consisting of C₂₋₈ alkyl,
R² is C₁ alkyl,
R³ is independently selected from the group consisting of H and C₁₋₈ alkyl,
R⁴ is independently selected from the group consisting of C₁₋₈ alkyl,
or a pharmaceutically acceptable salt thereof.

2. The compound of claim 1, wherein the pharmaceutically acceptable salt is selected from the group consisting of hydrochloride salt, succinate salt, fumarate salt, L-malate salt, ketoglutarate salt, and citrate salt.

3. The compound of claim 1, which is 2-ethyl-4,6-dimethyl-3-hydroxypyridine, succinic acid (1:1), or 2-ethyl-4,5,6-trimethyl-3-hydroxypyridine, succinic acid (1:1).

4. A pharmaceutical composition comprising the compound of formula (I) as depicted in claim 1, or a pharmaceutically acceptable salt thereof, and a pharmaceutically acceptable carrier.

5. A process for preparing the compound of formula (I) as depicted in claim 1, comprising the step of reacting a compound of formula (II) 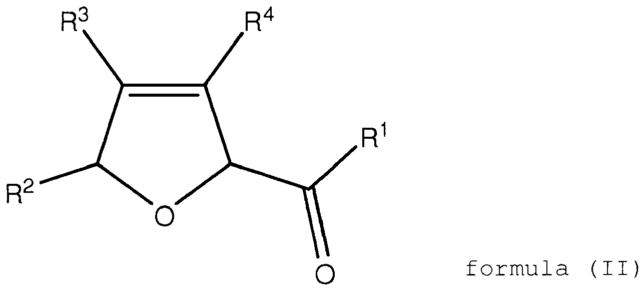 with ammonia, wherein
R¹ is selected from the group consisting of C₂₋₈ alkyl,
R² is C₁ alkyl,
R³ is independently selected from the group consisting of H and C₁₋₈ alkyl,
R⁴ is independently selected from the group consisting of C₁₋₈ alkyl.

6. The process of claim 5, wherein the compound of formula (I) is 2-ethyl-4,6-dimethyl-3-hydroxypyridine or 2-ethyl-4,5,6-trimethyl-3-hydroxypyridine.

## Patentansprüche

1. Verbindung der Formel (I): 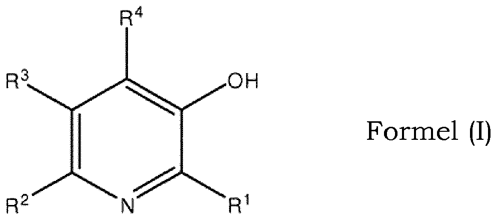 worin:
R¹ unter C₂₋₈-Alkylgruppen ausgewählt ist;
R² C₁-Alkyl ist;
R³ unabhängig unter H oder C₂₋₈-Alkylgruppen ausgewählt ist;
R⁴ unabhängig unter C₁₋₈-Alkylgruppen ausgewählt ist;
oder ein pharmazeutisch akzeptables Salz davon.

2. Verbindung nach Anspruch 1, worin das pharmazeutisch akzeptable Salz unter einem Hydrochloridsalz, einem Succinatsalz, einem Fumaratsalz, einem L-Malatsalz, einem Ketoglutaratsalz und einem Citratsalz ausgewählt ist.

3. Verbindung nach Anspruch 1, bei der es sich um 2-Ethyl-4,6-dimethyl-3-hydroxypyridin, Bernsteinsäure (1:1) oder 2-Ethyl-4,5,6-trimethyl-3-hydroxypyridin, Bernsteinsäure (1:1) handelt.

4. Pharmazeutische Zusammensetzung, die die Verbindung der Formel (I) wie in Anspruch 1 dargestellt oder ein pharmazeutisch akzeptables Salz davon und einen pharmazeutisch akzeptablen Träger enthält.

5. Verfahren zur Herstellung einer Verbindung der Formel (I) wie in Anspruch 1 dargestellt, das den Schritt umfasst, eine Verbindung der Formel (II): 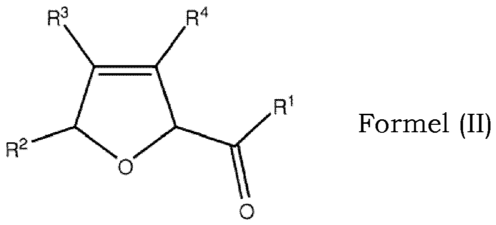 worin:
R¹ unter C₂₋₈-Alkylgruppen ausgewählt ist;
R² C₁-Alkyl ist;
R³ unabhängig unter H oder C₂₋₈-Alkylgruppen ausgewählt ist;
R⁴ unabhängig unter C₁₋₈-Alkylgruppen ausgewählt ist;
mit Ammoniak umzusetzen.

6. Verfahren nach Anspruch 5, worin die Verbindung der Formel (I) 2-Ethyl-4,6-dimethyl-3-hydroxypyridin oder 2-Ethyl-4,5,6-trimethyl-3-hydroxypyridin ist.

## Revendications

1. Composé de formule (I) 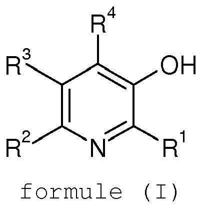 dans laquelle
R¹ est choisi parmi les radicaux alkyle en C₂ à C₈,
R² est un radical alkyle en C₁,
R³ est indépendamment choisi dans le groupe constitué par H et les radicaux alkyle en C₁ à C₈,
R⁴ est indépendamment choisi parmi les radicaux alkyle en C₁ à C₈,
ou un sel pharmaceutiquement acceptable d'un tel composé.

2. Composé selon la revendication 1, dans lequel le sel pharmaceutiquement acceptable est choisi dans le groupe constitué par le sel chlorhydrate, le sel succinate, le sel fumarate, le sel L-malate, le sel cétoglutarate, et le sel citrate.

3. Composé selon la revendication 1, qui est le sel d'acide succinique et de 2-éthyl-4,6-diméthyl-3-hydroxypyridine (1:1) ou le sel d'acide succinique et de 2-éthyl-4,5,6-triméthyl-3-hydroxypyridine (1:1).

4. Composition pharmaceutique comprenant le composé de formule (I) tel que défini dans la revendication 1, ou un sel pharmaceutiquement acceptable de celui-ci, et un véhicule pharmaceutiquement acceptable.

5. Procédé pour préparer le composé de formule (I) tel que défini dans la revendication 1, comprenant l'étape consistant à faire réagir un composé de formule (II) 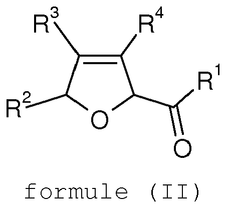 avec de l'ammoniac, où
R¹ est choisi parmi les radicaux alkyle en C₂ à C₈,
R² est un radical alkyle en C₁,
R³ est indépendamment choisi dans le groupe constitué par H et les radicaux alkyle en C₁ à C₈,
R⁴ est indépendamment choisi parmi les radicaux alkyle en C₁ à C₈.

6. Procédé selon la revendication 5, dans lequel le composé de formule (I) est la 2-éthyl-4,6-diméthyl-3-hydroxypyridine ou la 2-éthyl-4,5,6-triméthyl-3-hydroxypyridine.
